(19) Europäisches Patentamt
European Patent Office
Office européen des brevets

(11) Veröffentlichungsnummer: **0 282 542 B1**

(12) **EUROPÄISCHE PATENTSCHRIFT**

(45) Veröffentlichungstag der Patentschrift: **18.05.94**

(51) Int. Cl.5: **C07B 41/00**, C07B 43/00, C07B 37/00

(21) Anmeldenummer: **87906083.8**

(22) Anmeldetag: **16.09.87**

(86) Internationale Anmeldenummer: **PCT/EP87/00527**

(87) Internationale Veröffentlichungsnummer: **WO 88/02357 (07.04.88 88/08)**

# (54) VERFAHREN ZUR HERSTELLUNG VON ALKYLEN- BZW. RINGVERKNÜPFTEN VERBINDUNGEN.

(30) Priorität: **24.09.86 DE 3632410**

(43) Veröffentlichungstag der Anmeldung: **21.09.88 Patentblatt 88/38**

(45) Bekanntmachung des Hinweises auf die Patenterteilung: **18.05.94 Patentblatt 94/20**

(84) Benannte Vertragsstaaten: **CH DE LI**

(56) Entgegenhaltungen:
**EP-A- 119 756** **WO-A-87/04158**
**CA-A- 6 515 421** **DE-A- 3 139 130**
**GB-A- 2 107 733** **US-A- 4 405 488**

**Houben-Weyl Band III, 12a, 553, JOC 1985, 50, 5761-5765, Kontakfe (Darmstadt) 1988 (2) 15-28.**

(73) Patentinhaber: **MERCK PATENT GmbH**
**Postfach,**
**Frankfurter Strasse 250**
**D-64271 Darmstadt(DE)**

(72) Erfinder: **POETSCH, Eike**
**Am Buchwald 4**
**D-6109 Mühltal 6(DE)**
Erfinder: **REIFFENRATH, Volker**
**Pfungstädter Str. 31**
**D-6100 Darmstadt(DE)**

**EP 0 282 542 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

The Journal of Organic Chemistry, vol. 42, no. 10, 13 May 1977, Ei-ichi Negishi et al.: "Selective carbon-carbon bond formation via transition metalcatalysis. 3.1. A highly selective synthesis of unsymmetrical biaryls and diarylmethanes by the nickel- or palladium-catalyzed reaction of aryl- and benzylzinc derivatives with aryl halides" pages 1821-1823 see the whole document cited in the application.

Bulletin of the Chemical Society of Japan, vol. 56, no. 1, January 1983, The Chemical Society of Japan, T. Hayashi et al.: " Asymmetric cross-coupling of organozinc reagents with alkenyl bromides catalyzed by a chiral ferrocenylphosphine-palladium complex", pages 363-364, see the whole document cited in the application.

Journal of the American Chemical Society, Vol. 102, no. 9, 23 April 1980, American Chemical Society, Ei-ichi Negishi et al.: Palladium-catalyzed cross-coupling reaction of homoallylic or homopropargylic organozincs with alkenylhalides as a new selective route to 1,5-dienes and 1,5-enynes, pages 3298-3299 see the whole document.

## Beschreibung

Die Erfindung betrifft ein Verfahren zur Herstellung von Alkylen- bzw. ringverknüpften Verbindungen, insbesondere von Verbindungen der Formel I

$R^1$-$(A^0)_k$-$(Z^1$-$A^1)_m$-$Z^0$-$(E)_n$-$(Q)_o$-$(Z^2$-$A^2)_p$-$R^2$ I

worin

| | |
|---|---|
| $R^1$ und $R^2$ | jeweils unabhängig voneinander H oder Alkyl mit 1-18 C-Atomen, worin auch eine oder zwei nicht benachbarte $CH_2$-Gruppen durch -O-, -CO-, -O-CO-und/oder -CO-O- ersetzt sein können, CN oder Halogen, |
| $A^0$ und $A^2$ | jeweils unabhängig voneinander eine 1,4-Cyclohexylengruppe, worin auch eine oder zwei nicht benachbarte $CH_2$-Gruppen durch -O- und/oder -S-ersetzt sein können und/oder die in 1-Stellung durch $R^3$ substituiert sein kann, eine unsubstituierte oder ein- oder zweimal durch $R^3$ substituierte 1,4-Phenylengruppe, worin auch eine oder mehrere CH-Gruppen durch N ersetzt sein können, |
| $A^1$ | eine 1,4-Cyclohexylengruppe, die in 1-Stellung durch $R^3$ substituiert sein kann, |
| E | $CR^3=CH^4$ oder $CHR^3$-$CHR^4$, |
| $R^3$, $R^4$ | jeweils unabhängig voneinander H, Alkyl mit 1-6 C-Atomen, F, Cl, Br, $CF_3$, CN, |
| $Z^0$ | eine Einfachbindung, |
| $Z^1$ und $Z^2$ | jeweils -CO-O-, -O-CO-, $-CH_2CH_2-$, $-CHCN-CH_2-$, $-CH_2-CHCN-$, -CH=CH-, $-OCH_2-$, $-CH_2O-$, -CH=N-, -N=CH-, -NO=N-, -N=NO- oder eine Einfachbindung, |
| Q | eine unsubstituierte oder ein- oder zweimal druch $R^3$ substituierte 1,4-Phenylengruppe, worin auch eine oder mehrere CH-Gruppen durch N ersetzt sein können, |
| p | 0, 1 oder 2, |
| m | 1 oder 2, und |
| n, o und k | jeweils 0 oder 1 bedeuten, |

mittels Zinkorganylen.

Die bisher bekannten Verfahren zur Herstellung von Verbindungen der Formel I führen alle zu unbefriedigenden Ergebnissen. Hierfür sind vor allem schlechte Ausbeuten und z.T. schwer zugängliche Ausgangsverbindungen verantwortlich. So gelingt beispielsweise die Herstellung von 1-Cyano-2-fluor-4-(trans-4-heptylcyclohexyl)-benzol, einer Verbindung der Formel I, nach der EP-0 119 756 aus der entsprechenden 1-Acetylverbindung nur mit einer Ausbeute von ca. 1 % d.Th.

Auch die Herstellung von 1-Fluor-2-(trans-4-alkylcyclohexyl)-Verbindungennach der in US-4 405 499 offenbarten Methode erfordert erheblichen synthetischen Aufwand und liefert die gewünschten Produkte nur in unbefriedigenden Ausbeuten.

Die Herstellung von Cyclohexylzinkbromid aus Cyclohexylbromid und einer Zink-Kupfer-Verbindung ist aus N.N. Shabanova und G.G. Petukhov, Zh. Obshch. Khim. 36 (7), 1290-1292 (1966) (Russ.) bekannt.

Weiterhin sind Verfahren bekannt, die es erlauben, Organo-Zink-Verbindungen unter Übergangsmetallkatalyse mit Halogen-Verbindungen zu koppeln. Allerdings sind nur solche Kopplungen beschrieben, bei denen eine $\beta$-Eliminierung durch Verwendung von Aryl-, Benzyl-, Allyl-, Ethinyl- oder Methyl-Zink-Verbindungen ausgeschlossen ist (z.B. T. Hayashi et al., Bull. Chem. Soc. Japan 56, 363 (1983), E. Negishi et al., J. Am. Chem. Soc. 102, 3298 (1980), E. Negishi et al., J. Org. Chem. 42, 1821 (1977) oder M.T. Reetz et al., J. Chem. Soc., Chem. Comm. 1980, 1202).

Übergangsmetallkatalysierte Kreuzkupplungsreaktionen zur Synthese von Flüssigkristallen werden weiterhin in den Dokumenten GB-A-2 107 733, DE-A 3 139 130 und WO-A-87/04158 beschrieben.

Aufgabe der vorliegenden Erfindung war es, ein Herstellungsverfahren für die Verbindungen der Formel I zu finden, das die beschriebenen Nachteile der bisherigen Verfahren nicht oder nur in geringem Maß aufweisen.

Überraschenderweise wurde gefunden, daß zinkorganische Verbindungen in Gegenwart eines Metallkatalysators hervorragend zur Herstellung von Verbindungen der Formel I geeignet sind.

Gegenstand der Erfindung ist somit ein Verfahren zur Herstellung von Verbindungen der Formel I, dadurch gekennzeichnet, daß man eine Verbindung der Formel IV,

$R^1$-$(A^0)_k$-$(Z^1$-$A^1)_m$-$Z^0$-X' IV

worin $R^1$, $A^0$, $A^1$, $Z^0$, k und m die angegebene Bedeutung besitzen und X' Chlor, Brom oder Iod bedeutet, in Gegenwart von metallischem Lithium unter Anwendung von Ultraschall mit einem Zinkhalogenid behandelt

und die dabei erhaltene Organozinkverbindung der Formel II,

$R^1$-$(A^0)_k$-$(Z^1$-$A^1)_m$-$Z^0$-ZnY II

worin $R^1$, $A^0$, $A^1$, $Z^0$, k und m die für Formel I angegebene Bedeutung besitzen und Y Cl, Br, I oder ein Rest $R^1$-$(A^0)_k$-$(Z^1$-$A^1)_m$-$Z^0$- mit der voranstehenden Bedeutung ist, mit einer Verbindung der Formel III,

X-$(E)_n$-$(Q)_o$-$(Z^2$-$A^2)_p$-$R^2$ III

worin $R^2$, E, $A^2$, $Z^2$, Q, n, o und p die für Formel I angegebene Bedeutung besitzen und X Br, I, $OSO_2$-$(CF_2)_{0-10}$-$CF_3$ bedeutet, unter Zusatz einer Metallverbindung koppelt, mit der Maßgabe, daß $Z^2$ -$CH_2CH_2$-, -CHCN-$CH_2$-, -$CH_2$-CHCN, -CH=CH- oder eine Einfachbindung bedeutet, falls n + o = 0 ist.

Das Gelingen einer derartigen stereoselektiven Kopplung aliphatischer Zinkorganyle mit aromatischen, vinylischen, aliphatischen oder cycloaliphatischen Halogenverbindungen oder Sulfonaten war um so überraschender, als bei bisher bekannten C-C-Kopplungsreaktionen eine $\beta$-Eliminierung aus der der Kopplungsposition benachbarten CH-Gruppe entweder bei der Herstellung des Metallorganyls oder aus dem Metallorganyl selbst als den sterischen Verlauf und die Ausbeute stark beeinflussender Nebenreaktion auftritt.

Das erfindungsgemäße Verfahren geht von den leicht zugänglichen, metallierbaren Verbindungen der Formel IV aus

$R^1$-$(A^0)_k$-$(Z^1$-$A^1)_m$-$Z^0$X' IV

worin $R^1$, $A^0$, $A^1$, $Z^0$, k und m die für Formel I angegebene Bedeutung besitzen und X' Chlor, Brom, Iod bedeutet.

Diese werden nach bekannten Verfahren, wie sie z.B. in Houben-Weyl, Methoden der organischen Chemie, Georg-Thieme-Verlag, Stuttgart, Bd. 13/2a beschrieben sind, in die entsprechende Zinkorganyle der Formel II überführt. Die angewandten Methoden werden dabei zweckmäßig der Natur in den metallierbaren Verbindungen der Formel IV befindlichen funktionellen Gruppen angepaßt. So gelingt beispielsweise durch Ummetallierung von Alkalimetallderivaten in der von Suche et al., Tetrahedron Lett. 1984, 3463, beschriebenen Weise die Herstellung von sonst nur schwer zugänglichen Zinkorganylen.

Die Umsetzung der metallierbaren Verbindungen der Formel IV erfolgt vorzugsweise in einem inerten Lösungsmittel, beispielsweise Ethern wie Diethylether, Dimethoxyethan, Tetrahydrofuran oder Dioxan, Kohlenwasserstoffen wie Hexan, Cyclohexan, Benzol oder Toluol oder Gemischen dieser Lösungsmittel mit einem Zinkhalogenid wie Zinkchlorid, Zinkbromid oder Zinkiodid in Gegenwart metallischen Lithiums bei Temperaturen von -100° bis +100 °C, vorzugsweise bei -20 °C bis +50 °C. Besonders vorteilhaft ist dabei die Anwendung von Ultraschall. Unter diesen Bedingungen ist die Umwandlung der metallierbaren Verbindungen der Formel IV in die Zinkorganyle der Formel II gewöhnlich in etwa 10 bis 60 Minuten beendet.

Verbindungen der Formel III, worin $R^1$, $A^0$, $A^1$, $Z^0$, $Z^1$, k und m die in Anspruch I angegebene Bedeutung besitzen, wobei Cyclohexylzinkbromid ausgeschlossen wird, sind ebenfalls Gegenstand der Erfindung.

Anschließend fügt man der Lösung des Zinkorganyls der Formel II die Kopplungskomponente der Formel III und einen geeigneten Metallkatalysator zu, wobei eine selektive Kopplung zu den Verbindungen der Formel I erfolgt.

Zweckmäßigerweise wendet man dabei die gleichen wie für die Herstellung der Zinkorganylse der Formel II voranstehend genannten Lösungsmittel an bei den angegebenen Temperaturen. Die erforderlichen Reaktionszeiten betragen in der Regel zwischen etwa 1 und 50 Stunden.

Als Ausgangsverbindungen werden metallierbare Verbindungen der Formel IV und Verbindungen der Formel III eingesetzt. Als einsetzbare Halogenverbindungen kommen alle Halogenide, die sich in ein Zinkorganyl umwandeln lassen, in Frage. Vorzugs-Die nachfolgenden Formeln IVa bis IVf stellen eine Gruppe von ganz besonders bevorzugten Ausgangsverbindungen dar, wobei die Cyclohexylgruppen auch in 1-Stellung durch F, Cl, $CF_3$, CN oder $C_1$-$C_4$ Alkyl substituiert sein können:

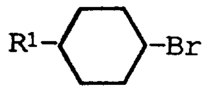

IVa

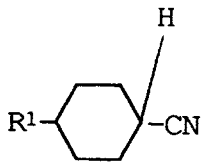

IVb

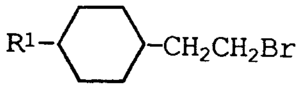

IVc

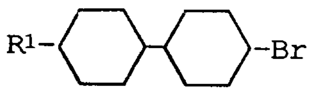

IVd

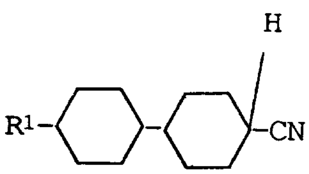

IVe

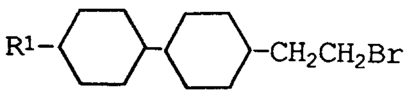

IVf

$R^1$ bedeutet darin vorzugsweise eine Alkylgruppe mit 1-18 C-Atomen, worin auch eine oder zwei nicht benachbarte $CH_2$-Gruppen durch -O- und/oder -CO- und/oder -CO-O-ersetzt sein können.

In den Formeln I, II, III und IV bedeuten $R^1$ bzw. $R^2$ vorzugsweise geradkettiges oder verzweigtes Alkyl mit 1-18 C-Atomen, vorzugsweise mit 2-10 C-Atomen, und bedeuten demnach bevorzugt Ethyl, Propyl, Butyl, Pentyl, Hexyl, Heptyl, Octyl, Nonyl oder Decyl, ferner auch Methyl, Undecyl, Dodecyl, Tridecyl, Tetradecyl oder Pentadecyl. Bevorzugt sind auch verzweigte Reste wie Isopropyl, 2-Butyl, Isopentyl, 1-Methylpentyl, 2-Methylpentyl, 1-Methylhexyl oder 1-Methylheptyl. $R^1$ bzw. $R^2$ bedeutet vorzugsweise auch Ethoxy, Propoxy, Butoxy, Pentoxy, Hexoxy, Heptoxy, Octoxy, Nonoxy und Decoxy, ferner auch Methoxy, Undecoxy, Dodecoxy, Tridecoxy, Tetradecoxy oder Pentadecoxy oder auch Isopropoxy, Isopentoxy, 2-Butoxy oder 1-Methylpentoxy.

Für die Kopplung mit den Zinkorganylen der Formel II verwendbar sind alle Verbindungen der Formel III. Vorzugsweise werden die Bromide (X = Br) eingesetzt, ferner auch die Chloride (X = Cl) und Perfluoralkansulfonate (X = $OSO_2$-$(CF_2)_{0-10}$-$CH_3$). Unter den Perfluoralkansulfonaten besonders bevorzugt sind die Trifluormethan-, Perfluorethan- und Perfluorpropansulfonate.

Die nachfolgenden Formeln IIIa bis IIIo stellen eine Gruppe von ganz besonders bevorzugten Ausgangsverbindungen dar, wobei die aromatischen Gruppen auch durch F, Cl, CN oder $CH_3$, die Cyclohexylgruppen auch in 1-Stellung durch F, Cl, $CF_3$, CN oder $C_1$-$C_4$ Alkyl substituiert sein können:

Br-CH = CH-$R^2$ IIIa

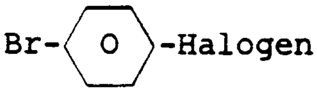

IIIb

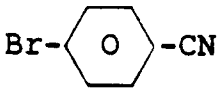

IIIc

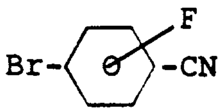

IIId

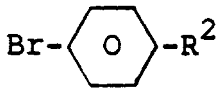

IIIe

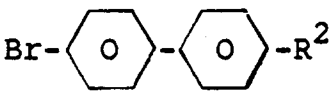

IIIf

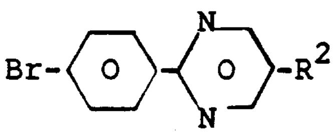

IIIg

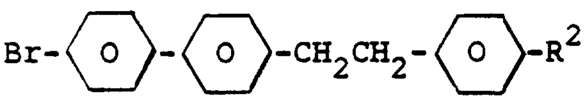

IIIh

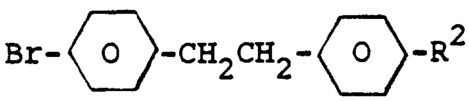

IIIi

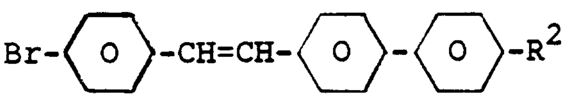

IIIj

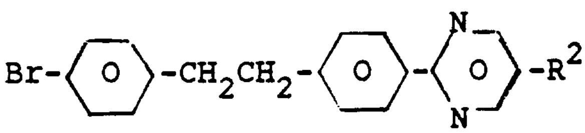

IIIk

Br-$R^2$ IIII

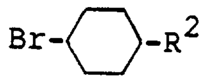

IIIm

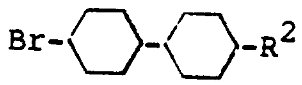

IIIn

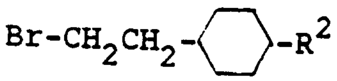

IIIo

$R^2$ bedeutet darin vorzugsweise eine Alkylgruppe mit 1-15 C-Atomen, worin auch eine oder zwei nicht benachbarte $CH_2$-Gruppen durch -O- und/oder -CO- und/oder -CO-O-ersetzt sein können.

Als metallische Katalysatoren werden vorzugsweise solche des Palladiums und/oder Nickels verwendet. Zur Bildung geeigneter Komplexe befähigte Liganden finden sich beispielsweise in der Gruppe der Phosphine wie Triphenylphosphin, Tritolylphosphin, Tris-(4-dimethylaminophenyl)-phosphin oder 1,2-Bis-(diphenylphosphino)-ethan oder 1,1'-Bis-(diphenylphosphino)-ferrocen oder der Diketone wie Acetylaceton oder Octafluoracetylaceton.

Es können auch Salze der vorstehend genannten Metalle Verwendung finden, wie beispielsweise $LiPdCl_3$ oder entsprechende Nickelsalze. Weiterhin eignen sich auch gemischte Komplexe wie Bis-(triphenylphosphin)-nickeldichlorid.

Die Verwendung von Tetrakis-(triphenylphosphin)-palladium oder von [1,1'Bis-(Diphenylphosphin)-ferrocenyl]-palladium als Katalysator stellt eine besonders bevorzugte Ausführungsform der vorliegenden Erfindung dar.

Die als Ausgangsverbindungen eingesetzten Halogenverbindungen der Formel IV sind bekannt oder können nach bekannten Methoden, wie beispielsweise in Houben-Weyl, Methoden der Organischen Chemie, Bd. 5/3 und 5/4 beschrieben, hergestellt werden. So ist es beispielsweise möglich, Alkohole der Formel IV, in denen X' für eine Hydroxylgruppe steht, mit einem Phosphortrihalogenid oder mit Triphenylphosphin/Tetrahalogenmethan in die entsprechenden Halogenide zu überführen.

Ebenfalls bekannt oder nach bekannten Methoden erhältlich sind die als Kopplungskomponenten einzusetzenden Verbindungen der Formel III.

Beispielsweise lassen sich Halogenide der Formel III, worin X Br oder I, n 0 und Q einen Aromaten bedeutet, dadurch erhalten, daß man in einem entsprechenden Diazoniumsalz die Diazoniumgruppe durch Br oder I ersetzt.

Perfluoralkylsulfonate der Formel III, worin X $OSO_2$-$(CF_2)_{0-10}$-$CF_3$ bedeutet, können durch Veresterung entsprechender Alkohole der Formel III, worin X eine Hydroxylgruppe bedeutet, mit Perfluoralkansulfonsäuren oder ihren reaktiven Derivaten hergestellt werden. Die entsprechenden Perfluoralkansulfonsäuren sind bekannt.

Als reaktionsfähige Derivate der genannten Perfluoralkansulfonsäure eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

In der vorliegenden Erfindung steht somit ein sehr vorteilhaftes Verfahren zur einfachen, in hohen Ausbeuten verlaufenden, stereoselektiven Herstellung von Verbindungen der Formel I zur Verfügung.

Die Verbindungen der Formel I eignen sich zum Einsatz als flüssigkristalline Materialien, wie Z.B. in DE-32 17 597 und DE-26 36 684 offenbart, oder können als Zwischenprodukte für die Herstellung weiterer flüssigkristalliner Verbindungen vervendet werden.

Die folgenden Beispiele sollen die Erfindung näher erläutern, ohne sie zu begrenzen. Fp = Schmelzpunkt; K = kristallin; N = nematisch, I = isotrop. Vor- und nachstehend bedeuten Prozentangaben Gewichtsprozent.

Übliche Aufarbeitung bedeutet im folgenden: Mit schwach saurem Wasser und Toluol extrahieren, Trocknen der organischen Phase, Eindampfen und Reinigen durch Chromatographie.

Beispiel 1

5,75 g cis-4-(trans-4'-Propylcyclohexyl)-cyclohexylbromid (hergestellt aus dem entsprechenden trans-Alkohol mit Triphenylphosphin/Brom in Acetonitril in für die Bromierung von Alkoholen bekannter Weise) werden in einem Lösungsmittelgemisch aus 40 ml Toluol und 10 ml Tetrahydrofuran mit 2,3 g Zinkbromid und 0,28 g dünn gehämmerten Lithiumscheiben in einem mit Eiswasser gefüllten Ultraschallbad so lange dem Ultraschall ausgesetzt, bis kein Lithium mehr erkennbar ist. Die dabei gebildete schwarze Suspension wird mit 0,16 g Tetrakis(triphenylphosphin)-palladium(0) und 4 g 4-Brom-2-fluorbenzonitril versetzt und bei entferntem Ultraschallbad 18 h bei Zimmertemperatur gerührt. Darauf wird mit schwach saurem Wasser und Toluol extrahiert, die organische Phase über $MgSO_4$ getrocknet und eingedampft. Der Eindampfrückstand ergibt nach Chromatographie über Kieselgel mit Petrolether/ Diethylether 9:1 und Kristallisation aus Ethanol 3,5 g 4-Cyano-3-fluor-1-[trans-4'-(trans-4"-propylcyclohexyl)-cyclohexyl]benzol (54 % d.Th.) vom Schmelzpunkt 54,3 °C und Klärpunkt 203 °C.

Auf analoge Weise erhält man aus 6,06 g (0,02 Mol) cis4-(trans-4'-Butylcyclohexyl)-cyclohexylbromid und 5,4 g (0,02 Mol) 4-Trifluormethansulfonyloxy-2-fluorbenzonitril 2,3 g 4-Cyano-3-fluor-1-[trans-4'-(trans-4"-butylcyclohexyl)-cyclohexyl]-benzol; K/N 67°, N/I 197,4°.

Analog lassen sich die folgenden Verbindungen darstellen (die angegebenen Übergangstemperaturen beziehen sich auf den Übergang kristallin/nematisch (K/N) und nematisch/isotrop (N/I).

**4-[trans-4'-(trans-4"-Alkylcyclohexyl)-cyclohexyl]-halogenbenzole**

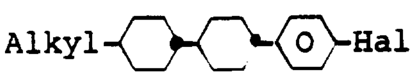

| Alkyl | Hal | K/N (°C) | N/I (°C) |
|---|---|---|---|
| $H_7C_3$ | F | 87,9 | 158,5 |
| $H_9C_4$ | F | 79,5 | 152,1 |
| $H_{13}C_6$ | Cl | 57,8 | 173,2 |

4-[trans-4'-(trans-4''-Alkylcyclohexyl)-cyclohexyl]-alkoxybenzole und -alkylbenzole

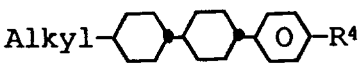

| Alkyl | $R^4$ | K/N (°C) | N/I (°C) |
|---|---|---|---|
| $H_7C_3$ | $OCH_3$ | 79,5 | 211 |
| $H_9C_4$ | $OC_6H_{13}$ | 70,0 | 186,3 |
| $H_7C_3$ | $CH_3$ | 64,6 | 179,8 |
| $H_{11}C_5$ | $C_3H_7$ | 48,6 | 181,0 |
| $H_7C_3$ | $C_5H_{11}$ | < 20 | 172,0 |

4-[trans-4'-(trans-4''-Alkylcyclohexyl)-cyclohexyl]-halogenbenzole

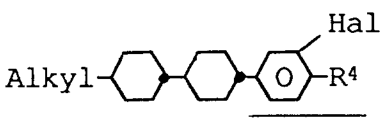

| Alkyl | Hal | $R^4$ | K/N (°C) | N/I (°C) |
|---|---|---|---|---|
| $H_7C_3$ | F | H | 54,5 | 92,3 |
| $H_9C_4$ | Cl | H | 28,3 | 39,8 |
| $H_7C_3$ | F | $COCH_3$ | 88 | 293 |

| | | | | |
|---|---|---|---|---|
| $H_9C_4$ | F | CN | 67 | 197,4 |

trans-4-Alkylcyclohexylarylderivate

Alkyl-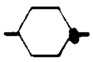-B-$R^4$

| Alkyl | B | $R^4$ | K/N (°C) | N/I (°C) |
|---|---|---|---|---|
| $H_7C_3$ | 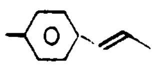 | 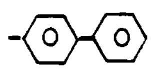-$C_7H_{15}$ | | |
| $H_7C_3$ | 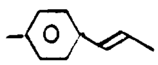 | 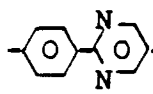-$C_7H_{15}$ | 106 | 169 |
| $H_7C_3$ | 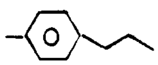 | 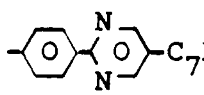-$C_7H_{15}$ | | |
| $H_7C_3$ | 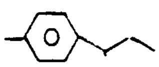 | 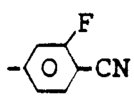-CN | | |
| $H_9C_4$ | 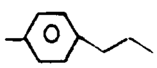 | 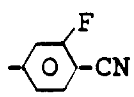-CN | | |
| $H_{11}C_5$ | 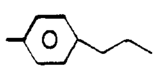 | 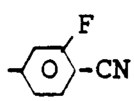-CN | | |
| $H_7C_3$ | 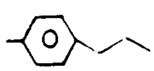 | 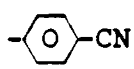-CN | | |
| $H_{15}C_7$ | 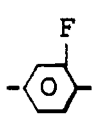 | -CN | 19 | 21 |

4-[trans-4'-(trans-4"-Alkylcyclohexyl)-cyclohexyl]-arylderivate

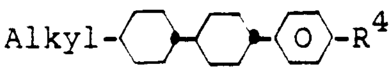

| Alkyl | $R^4$ | K/N (°C) | N/I (°C) |
|---|---|---|---|
| $C_3H_7$ | $COCH_3$ | 81 | 115 |
| $C_2H_5$ | CN | 76 | 195 |
| $C_3H_7$ | CN | 55 | 184 |
| $C_5H_{11}$ | 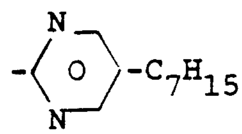 | 200 | 270 |

Beispiel 2

4,68 g trans-4-(trans-4'-Pentylcyclohexyl)-cyclohexancarbon-säurenitril werden in 7,5 ml Tetrahydrofuran gelöst und bei -75° bis -65 °C zu einer Lösung von Lithiumdiisopropylamid getropft, die in 7,5 ml Tetrahydrofuran aus 2,7 ml Diisopropylamin und 12,5 ml 15%iger Hexanlösung von Butyllithium bereitet wird. Nach der Zugabe wird noch 30 Minuten nachgerührt, dann werden unter weiterem Rühren 1,26 g $ZnCl_2$, gelöst in 10 ml Tetrahydrofuran, zugetropft. Es wird bei -50 °C eine halbe Stunde nachgerührt, dann werden 50 mg 1,2-Bisdiphenylphosphinoethano-Nicheldichlorid und 3,27 g 4-Brombenzonitril, gelöst in 15 ml Tetrahydrofuran, zugesetzt und nach Entfernung der Kühlung 72 Stunden bei Zimmertemperatur gerührt. Darauf wird mit Eiswasser zersetzt, eingedampft, mit Wasser/Petrolether extrahiert, die Petroletherphase getrocknet, eingedampft und über Kieselgel mit Petrolether/Diethylether 9:1 chromatographiert. Die Hauptfraktion ergibt nach zweimaliger Umkristallisation aus Essigsäureethylester 0,92 g trans-4-(trans-4'-pentylcyclohexyl)-1-cyanocyclohexyl]-benzonitril. Die Verwendung von $NiCl_2$ $(PPh_3)_2$ unter sonst gleichen Bedingungen führt zu vergleichbaren Ausbeuten.

Schmelzpunkt 113,3 °C, Klärpunkt: 135,6 °C, $\Delta\epsilon$ = 2,3

Analog werden aus dem Zinkderivat des trans-4-(trans-4'-Pentylcyclohexyl)-cyclohexancarbonsäurenitrils erhalten:

| | Schmelzpunkt °C | Klärpunkt °C | Δε |
|---|---|---|---|
| 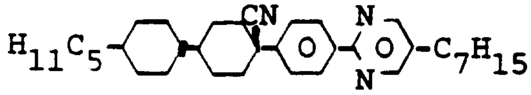 | 85,1 | 211,2 | -0,3 |
| 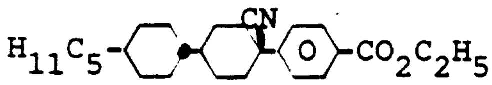 | 86,0 | 116,2 | -6,6 |

Beispiel 3

5,75 g cis-4-(trans-4'-Propylcyclohexyl)-cyclohexylbromid werden in 50 ml eines Lösuflgsmittelgemisches aus 40 ml Toluol und 10 ml Tetrahydrofuran mit 2,3 g Zinkbromid und 0,28 g (0,04 Mol) dünn gehämmerten Lithiumscheiben in einem gekühlten Ultraschallbad bei -10 °C bis 0 °C 2 Stunden beschallt. Nach dieser Zeit hat sich das Lithium aufgelöst und sich eine dünne schwarze Suspension gebildet. Es werden 0,16 g Tetrakis(triphenylphosphin)palladium(0) zugegeben und 3,2 g Vinylbromid zugetropft. Darauf wird 24 Stunden bei Zimmertemperatur gerührt, extraktiv mit Wasser und Toluol aufgearbeitet und der Toluoleindampfrückstand mit Naphtabenzin über Kieselgel filtriert. Nach dem Eindampfen werden 3,1 g eines Gemisches erhalten, das gemäß gaschromatograhischer Analyse zu über 40 % aus 1-[trans-4'-(Propylcyclohexyl)-trans-4''-cyclohexyl]-ethylen besteht.

Analog werden erhalten:

1-[trans-4'-(Pentylcyclohexyl)-trans-4''-cyclohexyl]ethylen trans-4-(trans-4-Pentylcyclohexyl)-1-octylcyclohexan

Analog erhält man nach anschließender Umkristallisation durch Umsetzung mit den entsprechenden Bromstyrolderivaten, deren Herstellung nach den in Houben-Weyl Band V/4 angegebenen Methoden gelingt, die nachstehend aufgeführten 1-Aryl-2[trans-4'-(alkylcyclohexyl)-trans-4''-cyclohexyl]-ethene:

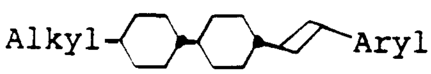

| Alkyl | Aryl | Schmelzpunkt | Klärpunkt |
|---|---|---|---|
| $C_4H_9$ | 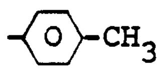 | 73 | 224 |
| $C_3H_7$ | 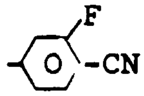 | 159 °C | 216 °C |
| $C_4H_9$ | 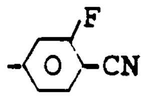 | 57 | 226 |

Beispiel 4

Aus 20,5 g eines 1:1 Gemisches der isomeren 4-Propylcyclohexylbromide wird nach der im Houben-Weyl Bd. 13/2a, S. 635 angegebenen Methode mittels einer Zink-Kupferlegierung die 4-Propylcyclohexylzinkbromidverbindung dargestellt. Diese ergibt in einer zu Beispiel 1 analogen Kopplungsreaktion mit 18,2 g Brombenzonitril 7,5 g 4-(trans-4-propylcyclohexyl)-benzonitril.

Analog werden die folgenden Verbindungen aus 4-substituierten Brombenzol-Derivaten erhalten:

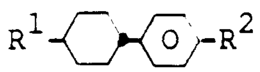

| $R^1$ | $R^2$ | Schmelzpunkt °C | Klärpunkt °C |
|---|---|---|---|
| Propyl | F | 32 | |
| Heptyl | F | 35 | |
| Heptyl | Propyloxy | 46 | |
| Pentyl | Butyloxy | 34 | 46 |
| Pentyl | Hexyloxy | 33 | 45 |
| Heptyl | Hexyloxy | 38 | 52 |

Beispiel 5

Ein Gemisch aus 21,0 g trans-4-(trans-4-Pentylcyclohexyl)-1-bromcyclohexan, 100 ml Toluol und 25 ml Tetrahydrofuran wird mit 7,7 g Zinkbromid und 1 g dünn gehämmerten Lithiumscheiben in einem mit Eiswasser gefüllten Ultraschallbad so lange dem Ultraschall ausgesetzt bis kein Lithium mehr erkennbar ist. Man fügt [1,1'-Bis-(Diphenylphosphin)ferrocenyl]palladium(0), hergestellt aus 0,57 g [(Diphenylphosphin)-ferrocenyl]-palladium-(II)-chlorid und 0,95 ml einer Lösung von Diisobutylaluminiumhydrid in Toluol (20%ig) und 19,3 g trans-4-(trans-4-Propylcyclohexyl)-1-bromcyclohexan in 50 ml Tetrahydrofuran hinzu, rührt 2 h bei Zimmertemperatur und 16 h in der Siedehitze. Nach üblicher Aufarbeitung erhält man trans-4-(trans-4-(trans-4-(trans-4-Propylcyclohexyl) cyclohexyl)-cyclohexyl)-1-pentylcyclohexan.

Beispiel 6

Ein Gemisch aus 103 g 4-trans-Propyl-1-bromcyclohexan, 120 ml Tetrahydrofuran und 480 ml Diethylether wird nach Beispiel 1 mit 57,5 g Zinkbromid und 7 g Lithium unter Ultraschall versetzt und mit einem Gemisch aus 71 g Bromethen, 70 ml Diethylether und 6 g Tetrakis(triphenylphosphin)palladium(0) umgesetzt. Nach üblichem Aufarbeiten erhält man trans-4-Propyl-1-vinylcyclohexan.

Beispiel 7

Ein Gemisch aus 1-Propylmagnesiumbromid, hergestellt aus 46,5 g 1-Brompropan und 13,4 g Magnesiumspänen und 200 ml Diethylether, wird mit 66,5 ml 1-Vinylcyclohex-3-en (aus Beispiel 8) tropfenweise versetzt.

Nach Zugabe von 1,45 ml Titantetrachlorid, Erhitzen und Abkühlen fügt man ein Gemisch von 34,1 g Zinkchlorid und 200 ml Tetrahydrofuran hinzu. Zu dem so erhaltenen Reaktionsgemisch gibt man ein

Gemisch aus 92,8 g 4-Brombenzonitril, 5,8 g [1,1'-Bis-(Diphenylphosphin)ferrocenyl]-palladium(0), erhalten nach Beispiel 7, 300 ml Diethylether und 200 ml Tetrahydrofuran. Nach üblichem Aufarbeiten erhält man 4-(2-Cyclohex-3-enylethyl)benzonitril.

## Patentansprüche

**1.** Verfahren zur Herstellung von Verbindungen der Formel I,

$R^1$-($A^o$)$_k$-($Z^1$-$A^1$)$_m$-$Z^o$-(E)$_n$-(Q)$_o$-($Z^2$-$A^2$)$_p$-$R^2$ I

worin

| | |
|---|---|
| $R^1$ und $R^2$ | jeweils unabhängig voneinander H oder Alkyl mit 1-18 C-Atomen, worin auch eine oder zwei nicht benachbarte $CH_2$-Gruppen durch -O-, -CO-, -O-CO- und/oder -CO-O- ersetzt sein können, CN oder Halogen |
| $A^o$ und $A^2$ | jeweils unabhängig voneinander eine 1,4-Cyclohexylengruppe, worin auch eine oder zwei nicht benachbarte $CH_2$-Gruppen durch -O-und/oder -S- ersetzt sein können und/oder die in 1-Stellung durch $R^3$ substituiert sein kann, eine unsubstituierte oder ein- oder zweimal durch $R^3$ substituierte 1,4-Phenylengruppe, worin auch eine oder mehrere CH-Gruppen durch N ersetzt sein können, |
| $A^1$ | eine 1,4-Cyclohexylengruppe, die in 1-Stellung durch $R^3$ substituiert sein kann, |
| E | $CR^3=CH^4$ oder $CHR^3$-$CHR^4$, |
| $R^3$, $R^4$ | jeweils unabhängig voneinander H, Alkyl mit 1-6 C-Atomen, F, Cl, Br, $CF_3$, CN, |
| $Z^o$ | eine Einfachbindung, |
| $Z^1$ und $Z^2$ | jeweils -CO-O-, -O-CO-, $-CH_2CH_2-$, $-CHCN-CH_2-$, $-CH_2-CHCN-$, -CH=CH-, $-OCH_2-$, $-CH_2O-$, -CH=N-, -N=CH-, -NO=N-, -N=NO- oder eine Einfachnindung, |
| Q | eine unsubstituierte oder ein- oder zweimal druch $R^3$ substituierte 1,4-Phenylengruppe, worin auch eine oder mehrere CH-Gruppen durch N ersetzt sein können, |
| p | 0, 1 oder 2, |
| m | 1 oder 2, und |
| n, o und k | jeweils 0 oder 1 bedeuten, |

dadurch gekennzeichnet, daß man eine Verbindung der Formel IV,

$R^1$-($A^o$)$_k$-($Z^1$-$A^1$)$_m$-$Z^o$-X' IV

worin $R^1$, $A^o$, $A^1$, $Z^o$, k und m, die angegebene Bedeutung besitzen und X' Chlor, Brom oder Iod bedeutet, in Gegenwart von metallischem Lithium unter Anwendung von Ultraschall mit einem Zinkhalogenid behandelt und die dabei erhaltene Organozinkverbindung der Formel II,

$R^1$-($A^o$)$_k$-($Z^1$-$A^1$)$_m$-$Z^o$-ZnY II

worin $R^1$, $A^o$, $A^1$, $Z^o$, $Z^1$, k und m die für Formel I angegebene Bedeutung besitzen und Y Cl, Br, I oder ein Rest $R^1$-($A^o$)$_k$-($Z^1$-$A^1$)$_m$-$Z^o$- mit der voranstehenden Bedeutung ist, mit einer Verbindung der Formel III,

X-(E)$_n$-(Q)$_o$-($Z^2$-$A^2$)$_p$-$R^2$ III

worin $R^2$, E, $A^2$, $Z^2$, Q, n, o und p die für Formel I angegebene Bedeutung besitzen und X Br, I, $OSO_2$-$(CF_2)_{0-10}$-$CF_3$ bedeutet, unter Zusatz einer Metallverbindung koppelt, mit der Maßgabe, daß $Z^2$ $-CH_2CH_2-$, $-CHCN-CH_2-$, $-CH_2-CHCN$, -CH=CH- oder eine Einfachbindung bedeutet, falls n + o = O ist.

**2.** Verfahren nach Anspruch 1, dadurch gekennzeichnet, daß man als Metallverbindung eine Verbindung des Palladiums und/oder Nickels verwendet.

**3.** Verfahren nach Anspruch 1, dadurch gekennzeichnet, daß man die Verbindungen der Formel II aus der Umsetzung der Verbindungen der Formeln

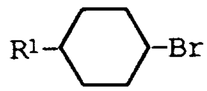

IVa

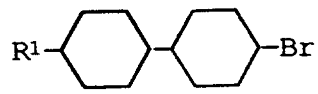

IVd

mit einem Zinkkhalogenid in Gegenwart metallischen Lithiums erhält.

4. Verbindungen der Formel II.

$R^1$-($A^o$)$_k$-($Z^1$-$A^1$)$_m$-$Z^o$-ZnY  II

worin $R^1$, $A^o$, $A^1$, $Z^o$, $Z^1$, k und m die in Formel I angegebene Bedeutung besitzen, wobei Cyclohexylzinkbromid ausgeschlossen wird.

## Claims

1. Process for the preparation of compounds of the formua I

$R^1$-($A^o$)$_k$-($Z^1$-$A^1$)$_m$-$Z^o$-(E)$_n$-(Q)$_o$-($Z^2$-$A^2$)$_p$-$R^2$  I

wherein

| | |
|---|---|
| $R^1$ and $R^2$ | in each case independently of one another are H or alkyl with 1-18 C atoms, wherein one or two non-adjacent $CH_2$ groups can also be replaced by -O-, -CO-, -O-CO- and/or -CO-O-, CN or halogen, |
| $A^o$ and $A^2$ | are in each case independently of one another a 1,4-cyclohexylene group, wherein one or two non-adjacent $CH_2$ groups can also be replaced by -O- and/or -S- and/or which can be substituted in the 1-position by $R^3$, or a 1,4-phenylene group which is unsubstituted or mono- or disubstituted by $R^3$, wherein one or more CH groups can also be replaced by N, |
| $A^1$ | is a 1,4-cyclohexylene group which can be substituted in the 1-position by $R^3$, |
| E | is $CR^3 = CR^4$ or $CHR^3$-$CHR^4$, |
| $R^3$, $R^4$ | are in each case independently of one another H, alkyl with 1-6 C atoms, F, Cl, Br, $CF_3$ or CN, |
| $Z^o$ | is a single bond, |
| $Z^1$ and $Z^2$ | are each -CO-O-, -O-CO-, $CH_2CH_2$-, -CHCN-$CH_2$-, -$CH_2$-CHCN-, -CH = CH-, -$OCH_2$-, -$CH_2O$-, -CH = N-, -N = CH-, -NO = N-, -N = NO- or a single bond, |
| Q | is a 1,4-phenylene group which is unsubstituted or mono- or disubstituted by $R^3$, wherein one or more CH groups can also be replaced by N, |
| p | is 0, 1 or 2, |
| m | is 1 or 2, and |
| n, o and k | are each 0 or 1, |

characterized in that a compound of the formula IV

$R^1$-($A^o$)$_k$-($Z^1$-$A^1$)$_m$-$Z^o$-X'  IV

wherein $R^1$, $A^o$, $A^1$, $Z^o$, k and m have the meaning given and X' is chlorine, bromine or iodine, is treated with a zinc halide in the presence of metallic lithium using ultrasound, and the resulting organozinc compound of the formula II

$R^1$-($A^o$)$_k$-($Z^1$-$A^1$)$_m$-$Z^o$-ZnY  II

wherein $R^1$, $A^o$, $A^1$, $Z^o$, $Z^1$, k and m have the meaning given for formula I and Y is Cl, Br, I or a radical $R^1$-($A^o$)$_k$-($Z^1$-$A^1$)$_m$-$Z^o$ with the above meaning, is coupled with a compound of the formula III

X-(E)$_n$-(Q)$_o$-($Z^2$-$A^2$)$_p$-$R^2$  III

wherein $R^2$, E, $A^2$, $Z^2$, Q, n, o and p have the meaning given for formula I and X is Br, I or $OSO_2$-$(CF_2)_{0-10}$-$CF_3$, with the addition of a metal compound, with the proviso that $Z^2$ is $-CH_2CH_2-$, $-CHCN-CH_2-$, $-CH_2-CHCN$, -CH = CH- or a single bond if n + o = 0.

**2.** Process according to Claim 1, characterized in that a compound of palladium and/or nickel is used as the metal compound.

**3.** Process according to Claim 1, characterized in that the compounds of the formula II are obtained from the reaction of the compounds of the formulae

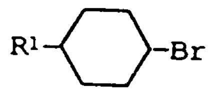

IVa

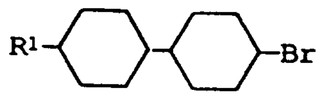

IVd

with a zinc halide in the presence of metallic lithium.

**4.** Compounds of the formula II

$R^1$-$(A^o)_k$-$(Z^1$-$A^1)_m$-$Z^o$-ZnY II

wherein $R^1$, $A^o$, $A^1$, $Z^o$, $Z^1$, k and m have the meaning given in formula I, with the exclusion of cyclohexylzinc bromide.

## Revendications

**1.** Procédé pour la préparation des composés de formule I

$R^1$-$(A^o)_k$-$(Z^1$-$A^1)_m$-$Z^o$-$(E)_n$-$(Q)_o$-$(Z^2$-$A^2)_p$-$R^2$ I

dans laquelle

| | |
|---|---|
| $R^1$ et $R^2$ | représentent chacun, indépendamment l'un de l'autre, H ou un groupe alkyle en C 1-C 18 dans lequel un ou deux groupes $CH_2$ non voisins peuvent être remplacés par -O-, -CO-, -O-CO- et/ou -CO-O, un groupe CN ou un halogène, |
| $A^1$ et $A^2$ | représentent chacun, indépendamment l'un de l'autre, un groupe 1,4-cyclohexylène dans lequel un ou deux groupes $CH_2$ non voisins peuvent être remplacés par -O- et/ou -S- et/ou qui peut être substitué en position 1 par $R^3$, un groupe 1,4-phénylène non substitué ou mono- ou di-substitué par $R^3$ et dans lequel un ou plusieurs groupes CH peuvent être remplacés par N, |
| $A^1$ | représente un groupe 1,4-cyclohexylène qui peut être substitué en position 1 par $R^3$, |
| E | représente $CR^3 = CH^4$ ou $CHR^3$-$CHR^4$, |
| $R^3$, $R^4$, | représentent chacun, indépendamment l'un de l'autre, H, un groupe alkyle en C 1-C 6, F, C1, Br, $CF_3$, CN, |
| $Z^o$ | représente une liaison simple, |
| $Z^1$ et $z^2$ | représentent chacun -CO-O, -O-CO-, $-CH_2CH_2$,$-CHCN-CH_2-$, $-CH_2-CHCN-$, -CH = CH-, $-OCH_2-$, $-CH_2O-$, -CH = N-, -N = CH-, -NO = N-, -N = NO- ou une liaison simple, |
| Q | représente un groupe 1,4-phénylène non substitué ou mono- ou di-substitué par $R^3$ et dans lequel un ou plusieurs groupes CH peuvent être remplacés par N, |
| p | est égal à 0, 1 ou 2, |
| m | est égal à 1 ou 2, et |
| n, o et k | sont chacun égaux à 0 ou 1, |

caractérisé en ce que l'on traite un composé de formule IV

$R^1$-$(A^o)_k$-$(Z^1$-$A^1)_m$-$Z^o$-X' IV

dans laquelle $R^1$, $A^o$, $Z^o$ , k et m ont les significations indiquées ci-dessus et X' représente le chlore, le brome ou l'iode, par un halogénure de zinc en présence de lithium métallique et sous application d'ultra-sons, ce qui donne un dérivé organozincique de formule II

$R^1\text{-}(A^o)_k\text{-}(Z^1\text{-}A^1)_m\text{-}Z^o\text{-}ZnY$ II

dans laquelle $R^1$, $A^o$, $A^1$, $Z^o$, $Z^1$, k et m ont les significations indiquées en référence à la formule I, et Y représente C1, Br, I ou un groupe $R^1\text{-}(A^o)_k\text{-}(Z^1\text{-}A^1)m\text{-}Z^o\text{-}$ dans lequel les symboles ont les significations indiquées ci-dessus,
qu'on condense avec un composé de formule III

$X\text{-}(E)_n\text{-}(Q)_o\text{-}(Z^2\text{-}A^2)_p\text{-}R^2$ III

dans laquelle $R^2$, E, $A^2$, $Z^2$, Q, n, o et p ont les significations indiquées en référence à la formule I et X représente Br, 1, $OSO_2\text{-}(CF_2)_{0-10}\text{-}CF_3$, en présence d'un composé métallique, sous réserve que, lorsque n + o = 0, $Z^2$ représente $\text{-}CH_2CH_2\text{-}$, $\text{-}CHCN\text{-}CH_2\text{-}$, $\text{-}CH_2\text{-}CHCN$, $\text{-}CH{=}CH\text{-}$ ou une liaison simple.

2. Procédé selon revendication 1, caractérisé en ce que le composé métallique utilisé est un composé du palladium et/ou du nickel.

3. Procédé selon revendication 1, caractérisé en ce que l'on obtient les composés de formule II par réaction des composés de formules

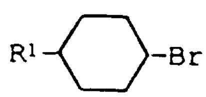

IVa

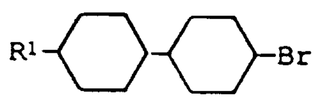

IVd

avec un halogénure de zinc en présence de lithium métallique.

4. Composés de formule II

$R^1\text{-}(A^o)_k\text{-}(Z^1\text{-}A^1)_m\text{-}Z^o\text{-}ZnY$ II

dans laquelle $R^1$, $A^o$ , $A^1$, $Z^o$, $Z^1$, k et m ont les significations indiquées en référence à la formule I, à l'exception du bromure de cyclohexyl-zinc.